# EUROPEAN PATENT SPECIFICATION

(11) **EP 1 787 574 B1**
(45) Date of publication and mention of the grant of the patent: **23.03.2016**
(21) Application number: 05780421.3
(22) Date of filing: 17.08.2005
(51) Int. Cl.: A61B 1/00, G06F 19/00, G06F 17/30

(54) **IMAGE DISPLAY DEVICE, IMAGE DISPLAY METHOD, AND IMAGE DISPLAY PROGRAM**
BILDANZEIGEVORRICHTUNG, BILDANZEIGEVERFAHREN UND BILDANZEIGEPROGRAMM
DISPOSITF DE VISUALISATION D'IMAGES, PROCÉDÉ DE VISUALISATION D'IMAGES ET PROGRAMME DE VISUALISATION D'IMAGES

(30) Priority: 18.08.2004 JP 2004238252
(43) Date of publication of application: 23.05.2007
(73) Proprietor: Olympus Corporation, Tokyo 151-0072 (JP)
(72) Inventor: HIRAKAWA, Katsumi, 2291103 (JP)
(74) Representative: von Hellfeld, Axel
(86) International application number: PCT/JP2005/015022
(87) International publication number: WO 2006/019120

(56) References cited:
- WO-A-02/073507
- JP-A- 2 289 226
- JP-A- 5 056 918
- JP-A- 2002 032 068
- US-A1- 2002 171 669
- US-A1- 2004 027 500

## Description

### TECHNICAL FIELD

The present invention relates to an image display apparatus, an image display method, and an image display program that sequentially display a plurality of stored images in an imaging order thereof.

### BACKGROUND ART

In recent years, in the field of endoscope, a swallowable capsule endoscope has been proposed. The capsule endoscope has an imaging function and a radio communication function. After the capsule endoscope is swallowed from the mouth of a subject (a human body) for observation (examination) and until it is naturally discharged, the capsule endoscope functions to move inside body cavities, for example, inside of organs such as a stomach and a small intestine according to peristalsis thereof, and to perform imaging of intra-subject images along with the movement at intervals of 0.5 second, for example.

Image data taken by the capsule endoscope while moving inside the body cavities are sequentially transmitted externally by radio communication and stored in a memory provided externally. Since the subject carries a receiving device having a radio communication function and a memory function, the subject can freely move during the period from swallowing the capsule endoscope and until it is discharged. After the capsule endoscope is discharged, doctors or nurses display images of organs on a display based on image data stored in the memory to perform diagnosis (for example, see Patent Document 1).

Patent Document 1: Japanese Patent Application Laid-open No. 2003-19111

Document US 2004/0027500 A1 concerns a system and a method for displaying an image stream. In one embodiment, it is disclosed to select images to be displayed substantially simultaneously based on spectral characteristics of each image display.

### DISCLOSURE OF INVENTION

### PROBLEM TO BE SOLVED BY THE INVENTION

In recent years, there has been proposed a technique for automatically extracting an image including a bleeding site from images taken by the capsule endoscope according to image processing. Conventionally, however, images stored in the memory are sequentially displayed on a display and when an extracted image is displayed, a predetermined marking is displayed. Since it is necessary for doctors or nurses to confirm images displayed on a display for confirmation of the extracted image one by one, a long time is spent as a confirming time of taken images. Also, there is a case that it is difficult to know a positional relationship between the displayed extracted image and an imaged site inside the subject, so that doctors or nurses cannot perform rapid diagnosis.

The present invention has been achieved in view of the problems in the conventional technique, and an object thereof is to provide an image display apparatus, an image display method, and an image display program that enable a user to conduct rapid image confirmation.

### MEANS FOR SOLVING PROBLEM

Some or all of the above problems are overcome by an image display apparatus having the features of claim 1, an image display method having the features of claim 6, and a computer readable recording medium having the features of claim 8. Further aspects of the invention are defined in the dependent claims.

### EFFECT OF THE INVENTION

According to the present invention, since there is provided the control unit that displays an extracted image extracted from a plurality of stored images under a preliminarily predetermined extraction condition, there can be achieved an effect that rapid confirmation of an extracted image by a user is made possible.

### BRIEF DESCRIPTION OF DRAWINGS

Fig. 1 is a schematic diagram showing an entire configuration of an intra-subject information acquiring system according to a first embodiment, which is an embodiment not forming part of the present invention;
Fig. 2 is a block diagram showing a schematic configuration of an image display apparatus according to the first embodiment;
Fig. 3 is a flowchart showing an image display process procedure performed by a control unit shown in Fig. 2;
Fig. 4 is a diagram showing one example of a display screen of a display unit shown in Fig. 2;
Fig. 5 is a diagram showing one example of a display screen of the display unit shown in Fig. 2;
Fig. 6 is a diagram showing one example of a display screen of the display unit shown in Fig. 2;
Fig. 7 is a block diagram showing a schematic configuration of an image display apparatus according to a second embodiment;
Fig. 8 is a flowchart showing an image display process procedure performed by a control unit shown in Fig. 7;
Fig. 9 is a diagram showing one example of a display screen of a display unit shown in Fig. 7;
Fig. 10 is a diagram showing one example of a display screen of the display unit shown in Fig. 7;
Fig. 11 is a block diagram showing a schematic configuration of an image display apparatus according to a third embodiment;
Fig. 12 is a flowchart showing an image display process procedure performed by a control unit shown in Fig. 11;
Fig. 13 is a flowchart showing a process procedure of image file creation shown in Fig. 12;
Fig. 14 is a diagram showing one example of a display screen of the display unit shown in Fig. 11;
Fig. 15 is a configuration diagram showing a configuration of a computer system using the first to third embodiments; and
Fig. 16 is a block diagram showing a configuration of a main body in the computer system shown in Fig. 15.

### EXPLANATIONS OF LETTERS OR NUMERALS

1 Subject
2 Receiving device
2a Receiving jacket
2b External device
4, 204, 304 Image display apparatus
5 Portable recording medium
20 Input unit
30 Display unit
40 Storage unit
50, 250, 350 Control unit
51 Image processor
52 Display controller
23 Cursor
24 Play button
25 All image display button
26 Extracted image display button
27 Next frame button
28 Previous frame button
29 File output button
253 Previous/next image extracting unit
354 Image file creator
100 Computer system
101 Main body
102 Display
102a Display screen
103 Keyboard
104 Mouse
105 Modem
106 Local area network or wide area network (LAN/WAN)
107 Public line
108 Flexible disk (FD)
109 CD-ROM
111 Another computer system (PC)
112 Server
113 Printer
121 CPU
122 RAM
123 ROM
124 Hard disk drive (HDD)
125 CD-ROM drive
126 FD drive
127 I/O interface
128 LAN interface

### BEST MODE(S) FOR CARRYING OUT THE INVENTION

Exemplary embodiments of an image display apparatus according to the present invention will be explained below in detail with reference to the accompanying drawings. Note that the invention is not limited to the embodiments. In addition, like parts are designated with like reference letters or numerals in described in the drawings. An image display apparatus in an intra-subject information acquiring system using a capsule endoscope will be explained as an example of the image display apparatus according to the embodiments.

### First Embodiment not forming part of the invention.

An image display apparatus according to a first embodiment will be explained first. The image display apparatus according to the first embodiment easily switches images to be displayed on a display screen to display either one of an extracted image or a taken image on the display screen.

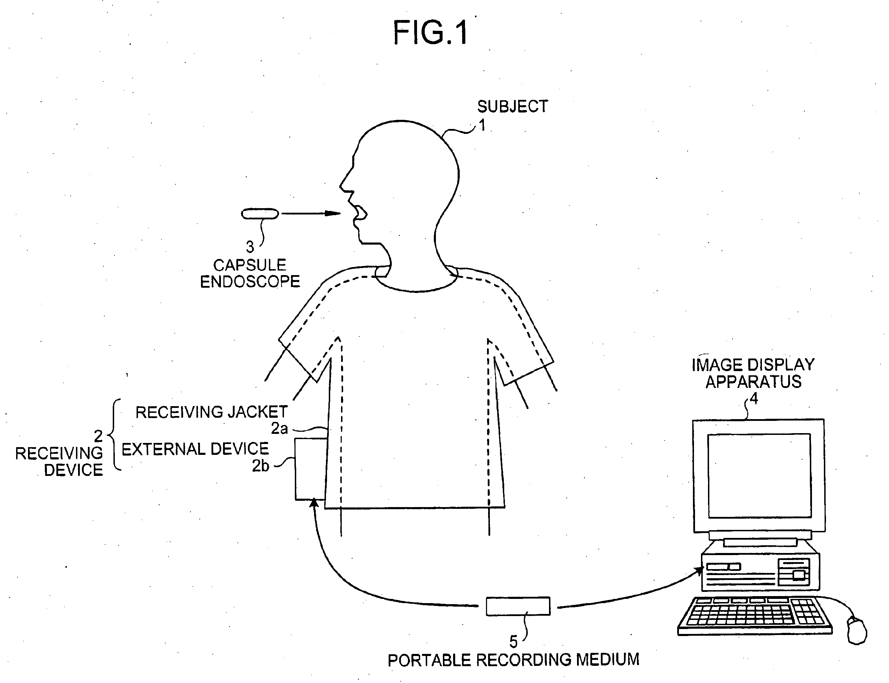

Fig. 1 is a schematic diagram showing an entire configuration of the intra-subject information acquiring system according to the present embodiment. As shown in Fig. 1, the intra-subject information acquiring system according to the embodiment includes a receiving device 2 having a receiving function and a capsule endoscope 3 that is inserted into a body of a subject 1 to image a body cavity image to perform data transmission to the receiving device 2. The intra-subject information acquiring system includes an image display apparatus 4 that displays a body cavity image based on data received by the receiving device 2 and a portable recording medium 5 for data transmissions between the receiving device 2 and the image display apparatus 4. The receiving device 2 includes a receiving jacket 2a worn by the subject 1 and an external device 2b performing processing of a radio signal or the like received via the receiving jacket 2a.

The image display apparatus 4 displays a body cavity image taken by the capsule endoscope 3 and includes a workstation or the like for performing image display based on data obtained by the portable recording medium 5. The image display apparatus 4 sequentially displays a plurality of still images taken by the capsule endoscope 3 in an imaging order thereof. Images sequentially displayed are called "pseudo moving image". The image display apparatus 4 displays an extracted image extracted by image processing based on a predetermined extraction condition.

The portable recording medium 5 is attachable to and detachable from the external device 2b and the image display apparatus 4, and it has a structure allowing output or recording of information at a loaded time to the both apparatuses. Specifically, the portable recording medium 5 is inserted into the external device 2b to record data transmitted from the capsule endoscope 3 while the capsule endoscope 3 is moving in the body cavities of the subject 1. After the capsule endoscope 3 is discharged from the subject 1, that is, after imaging inside the subject 1 is terminated, the portable recording medium 5 is taken out of the external device 2b to be inserted in the image display apparatus 4, and recorded data is read by the image display apparatus 4. Besides the configuration where transmissions of data between the external device 2b and the image display apparatus 4 are performed using the portable recording medium 5 such as a CompactFlash® memory, a configuration where the external device 2b and the image display apparatus 4 are connected by wire can be adopted.

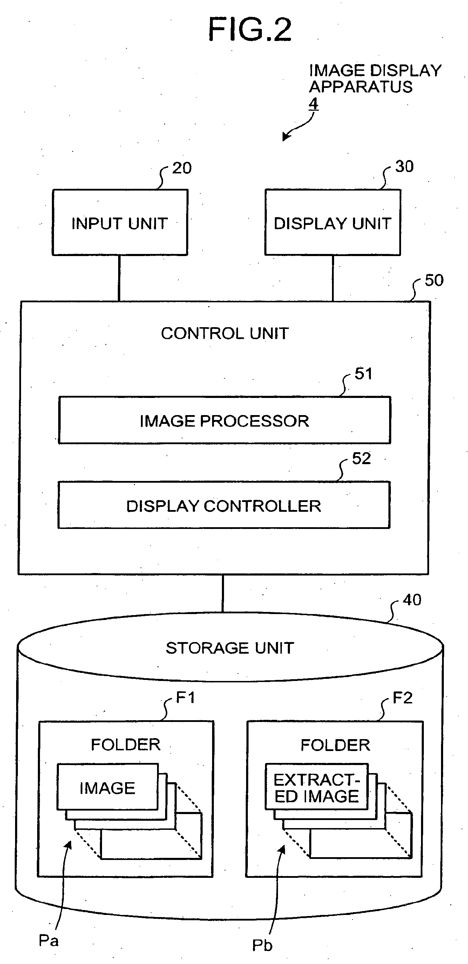

The image display apparatus 4 will be explained with reference to Fig. 2. Fig. 2 is a block diagram showing a schematic configuration of the image display apparatus 4 shown in Fig. 1. As shown in Fig. 2, the image display apparatus 4 includes an input unit 20, a display unit 30, a storage unit 40, and a control unit 50.

The input unit 20 is realized using a pointing device such as a keyboard or a mouse, and it inputs operation instruction information of the image display apparatus 4 and instruction information of a process performed by the image display apparatus 4 to output respective instruction information to the control unit 50. The input unit 20 inputs image instruction information instructing either one of a pseudo moving image or an extracted image extracted by an image processor 51 under a predetermined image condition as an image to be displayed on the display unit 30.

The display unit 30 is realized by a CRT display, a liquid crystal display, or the like, and it displays instruction information, an instruction result, or the like of the input unit 20. The display unit 30 displays either one of a pseudo moving image or an extracted image in a predetermined image display area under control of the control unit 50.

The storage unit 40 is realized, for example, by a hard disk device. Various images and the like are held in the storage unit 40, and an image group Pa imaged by the capsule endoscope 3 and an extracted image group Pb extracted by the image processor 51 in the control unit 50 are particularly stored in folders F1 and F2, respectively.

The control unit 50 controls respective processes and operations of the input unit 20, the display unit 30, and the storage unit 40. The control unit 50 includes the image processor 51 and a display controller 52. The image processor 51 extracts an image satisfying the predetermined extraction condition from a plurality of images stored in the folder F1. When an area corresponding to predetermined color information in a chromaticity diagram is included in an image so as to occupy at least a predetermined area, the image processor 51 extracts the image as an extracted image. For example, the image processor 51 determines presence/absence of a bleeding part based on presence/absence and a distribution of red color element in color information of an image to extract an image that has been determined to have a bleeding part as an extracted image. The display controller 52 controls an image display process in the display unit 30 and switches an image to be displayed in a predetermined image display area by the display unit 30 by the image instruction information inputted by the input unit 20 to either one of a pseudo moving image instructed in the image instruction information or the extracted image, to cause the display unit 30 to display the switched image in the predetermined image display area.

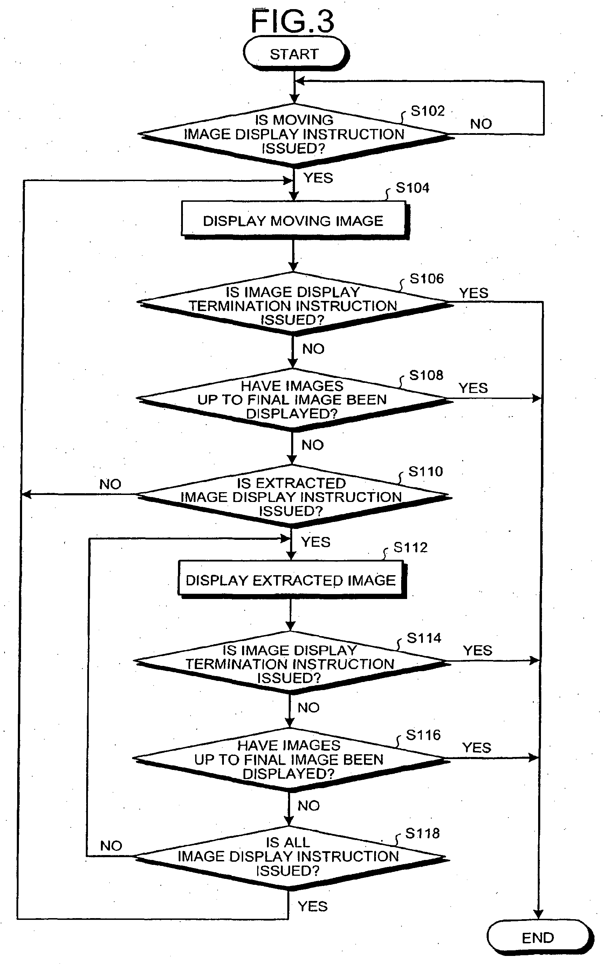

An image display process procedure in the control unit 50 will be explained next with reference to Fig. 3. In Fig. 3, the control unit 50 determines whether instruction information instructing pseudo moving image display from the input unit 20 is issued (step S102). The control unit 50 repeats determinations at step S102 until instruction information instructing pseudo moving image display is issued. When the control unit 50 determines that the instruction information instructing pseudo moving image display has been issued (step S102: Yes), the display controller 52 sequentially displays a plurality of images stored in the folder F1 in the image display area in an imaging order thereof, to perform the pseudo moving image display (step S104).

The display controller 52 determines whether instruction information instructing termination of the image display from the input unit 20 has been issued (step S106), and when the instruction information has been issued (step S106: Yes), the display controller 52 terminates the image display on the display unit 30. In this case, the pseudo moving image display in the image display area in the display unit 30 is terminated. On the other hand, when the display controller 52 determines that instruction information instructing termination of the image display from the input unit 20 has not been issued (step S106: No), the display controller 52 determines whether images up to a final image in the image group Pa have been displayed (step S108). When the display controller 52 determines that the images up to a final image in the image group Pa have been displayed (step S108; Yes), it terminates the pseudo moving image display in the image display area in the display unit 30.

On the other hand, when the display controller 52 determines that the images up to a final image in the image group Pa have not been displayed (step S108: No), the display controller 52 determines whether a display instruction of an extracted image to the display unit 30 has been issued based on presence/absence of the image instruction information from the input unit 20 (step S110). When the display controller 52 determines that the display instruction of an extracted image has not been issued (step S110: No), it proceeds to step S104, where the display controller 52 causes the display unit 30 to continue display of the pseudo moving image.

Further, when the display controller 52 determines that the display instruction of an extracted image has been issued (step S110: Yes), it switches an image to be displayed in the image display area in the display unit 30 to an extracted image to cause the display unit 30 to display an extracted image in the extracted image group Pb stored in the folder F2 in the storage unit 40 in the image display area (step S112).

Next, the display controller 52 determines whether instruction information instructing termination of the image display from the input unit 20 has been issued, like step S106 (step S114), and when the instruction information has been issued (step S114: Yes), the display controller 52 terminates the image display on the display unit 30. In this case, the extracted image display in the image display area in the display unit 30 is terminated. On the other hand, when the display controller 52 determines that instruction information instructing termination of the image display from the input unit 20 has not been issued (step S114: No), it determines whether the display unit 30 has displayed images up to the final image in the image group Pb which is an extracted image group (step S116). When the display controller 52 determines that the display unit 30 has displayed the images up to the final image in the image group Pb (step S116: Yes), it terminates the extracted image display in the image display area in the display unit 30.

On the other hand, when the display controller 52 determines that the display unit 30 has not displayed the images up to the final image in the image group Pb (step S116: No), it determines whether display instruction of a pseudo moving image of images stored in the folder F1, namely, display instruction of all images has been issued based on presence/absence of image instruction information from the input unit 20 (step S118). When the display controller 52 determines that the display instruction of all images has not been issued (step S118: No), it proceeds to step S112, where the display controller 52 causes the display unit 30 to continue display of the extracted image.

When the display controller 52 determines that the display instruction of all images has been issued (step S118: Yes), it proceeds to step S104, where it switches an image to be displayed in the image display area in the display unit 30 to a pseudo moving image and performs a pseudo moving image display, where the display controller 52 causes the display unit 30 to sequentially display images of the image group Pa stored in the folder F1 in the storage unit 40 in the image display area.

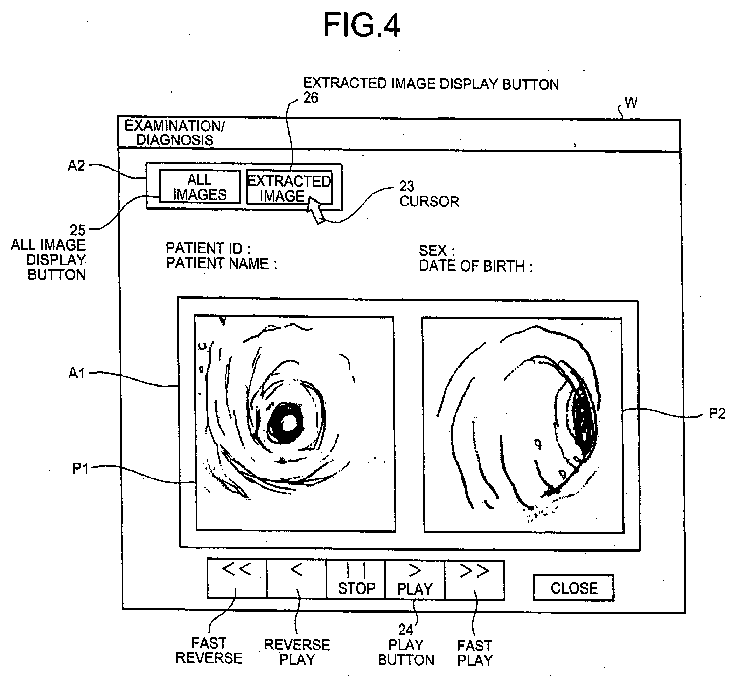

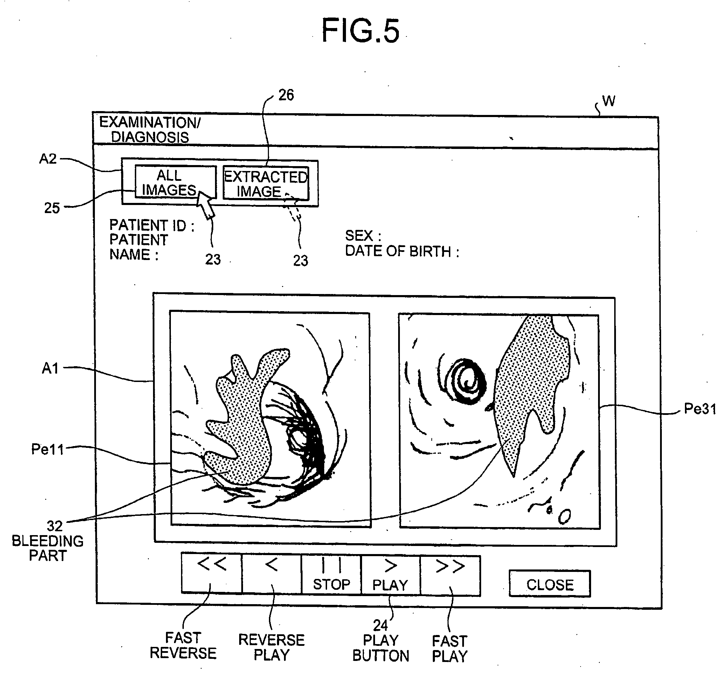

Next, switching between the pseudo moving image and the extracted image on the display screen in the display unit 30 will be explained with reference to Fig. 4 and Fig. 5. Fig. 4 and Fig. 5 are diagrams showing one example of the display screen in the display unit 30, where a window W is displayed on a display screen in the display unit 30. The window W roughly has an image display area A1 and an image instruction display area A2, where the image instruction display area A2 are displayed above the image display area A1, and various moving image display control buttons including a play button 24 are displayed below the image display area A1. Either one of a pseudo moving image or an extracted image instructed in the image instruction information is displayed in the image display area A1. Buttons displaying images displayed in the image display area A1 are respectively displayed in the image instruction display area A2. An all image display button 25 of the buttons displayed as "all images" corresponds to a display mode of a pseudo moving image, and an extracted image display button 26 displayed as "extracted image" corresponds to a display mode of an extracted image.

Switching of images to be displayed in the image display area will be explained with reference to Fig. 4. For example, a cursor 23 shown in Fig. 4 is moved onto the play button 24 according to an operation of the mouse of the input unit 20, and by a click of a left button on the mouse, instruction information instructing start of pseudo moving image display is inputted into the control unit 50 from the input unit 20. As a result, images P1 and P2 in the image group Pa stored in the folder F1 are displayed in the image display area A1 as a pseudo moving image according to an imaging order thereof. Unless a user selects the extracted image display button 26 in the image instruction display area A2, the images stored in the folder F1 are sequentially displayed in the image display area A1 in the imaging order as the pseudo moving image.

When a user wishes to change the image to be displayed in the image display area A1 from the pseudo moving image to the extracted image, the user moves the mouse of the input unit 20 to move the cursor 23 onto the extracted image display button 26 in the image instruction display area A2 and clicks the button. The input unit 20 selects the extracted image display button 26 according to the operation of the user to input image instruction information instructing an extracted image as the image to be displayed in the image display area A1 to the display controller 52. Thereafter, the display controller 52 switches the image to be displayed in the image display area A1 from the "all images" which is the pseudo moving image to the "extracted image" according to the image instruction information. The display controller 52 causes the display unit 30 to display the switched extracted image in the image display area A1. As a result, as shown in Fig. 5, images stored in the folder F2, for example, extracted images Pe11 and Pe31 including a bleeding part 32 are displayed in the image display area A1 in an imaging order thereof. Unless a user selects the all image display button 25 in the image instruction display area A2, extracted images stored in the folder F2 are sequentially displayed in the image display area A1 in the imaging order.

When an image to be displayed in the image display area A1 is switched to the pseudo moving image during display of the extracted images, the user moves the mouse to move the cursor 23 onto the all image display button 25 and clicks the left button on the mouse. As a result, the input unit 20 inputs image instruction information for switching the image to be displayed in the image display area A1 to the pseudo moving image display into the control unit 50. The display controller 52 switches the image to be displayed in the image display area A1 from the extracted images to the pseudo moving image to cause the display unit 30 to perform pseudo moving image display according to the image instruction information.

Thus, since the image display apparatus 4 according to the first embodiment causes the display unit 30 to display an extracted image preliminarily extracted from a plurality of stored images under a predetermined extraction condition, there can be achieved an effect that rapid confirmation of the extracted image by a user is made possible. Since the image display apparatus 4 according to the first embodiment can easily switch an image to be displayed in the image display area in the display unit 30 to either one of a pseudo moving image or an extracted image, the user can freely switch images to be displayed in the image display area. Therefore, according to image display apparatus 4 of the first embodiment, there can be achieved an effect that rapid and flexible image confirmation by a user can be realized.

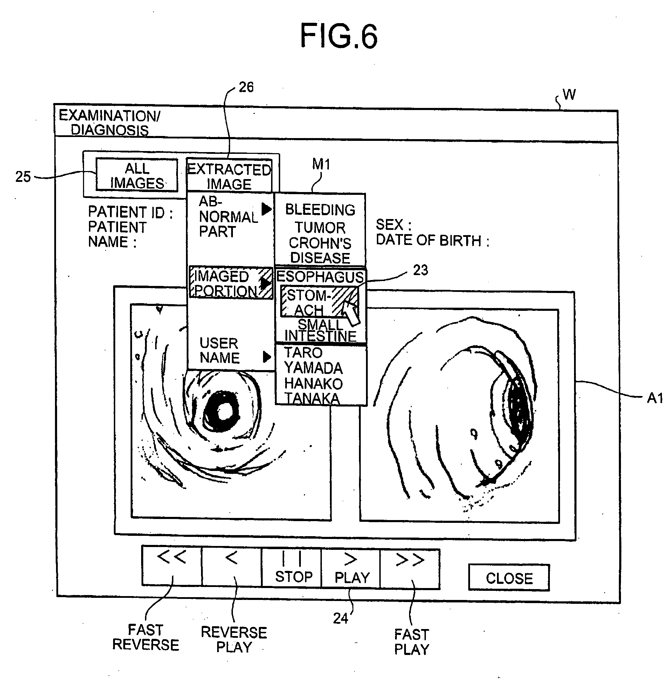

In the first embodiment, as shown in Fig. 5, the case that the extracted images Pe11 and Pe31 including the bleeding part 32 are displayed as the extracted images has been explained, however, extracted images to be displayed can be selected according to various extraction conditions. For example, the image processor 51 can be explained so as to extract a bleeding image including a bleeding part, a tumor image including tumor, a Crohn's disease image including a Crohn's disease site, and images that correspond to respective imaged portions such as an esophagus, a stomach, or a small intestine. Images respectively selected by each user can be included as the extracted images. In this case, as shown in Fig. 6, when a pseudo moving image display is switched to an extracted image display, the user selects the extracted image button 26. The user can select an extracted image extracted under a desired extraction condition by moving the cursor 23 onto an extraction condition to be displayed from respective extraction conditions shown in a menu M1 and by clicking on. As a result, an extracted image satisfying the extraction condition selected by the user is displayed in the image display area A1.

### Second Embodiment

Next, a second embodiment will be explained. In the second embodiment, since an image in the previous frame or the next frame of an extracted image is displayed together with the extracted image, a user can rapidly confirm a position of the displayed extracted image inside a subject.

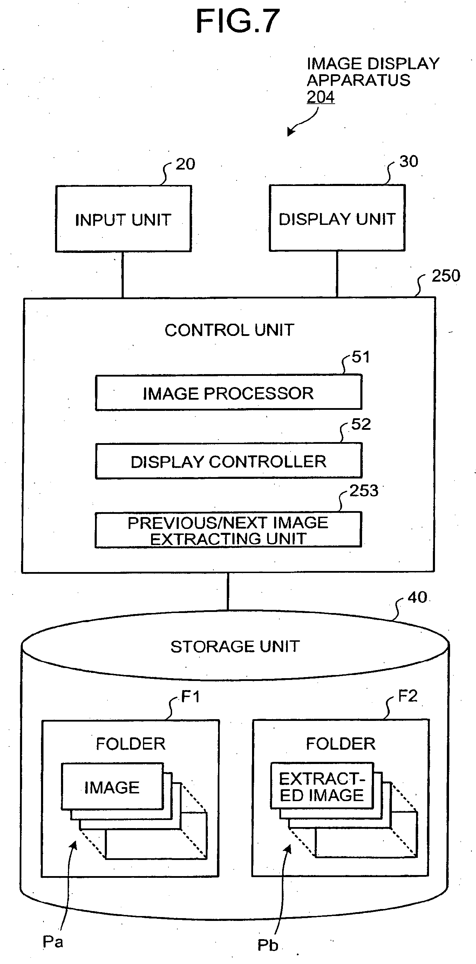

Fig. 7 is a block diagram showing a schematic configuration of an image display apparatus according to the second embodiment. As shown in Fig. 7, an image display apparatus 204 according to the second embodiment includes a control unit 250 further including a previous/next image extracting unit 253 in comparison with the control unit 50 according to the first embodiment. The input unit 20 inputs frame information instructing display of either image in the previous one frame or the next one frame of an extracted image displayed in the image display area in the display unit 30. The previous/next image extracting unit 253 extracts an image in the previous one frame or the next one frame of an extracted image displayed in the image display area from the image group Pa stored in the folder F1 in the storage unit 40 as the previous and next image based on the frame information inputted by the input unit 20. The display controller 52 causes the display unit 30 to display the image extracted by the previous/next image extracting unit 253 in a predetermined previous/next image display area.

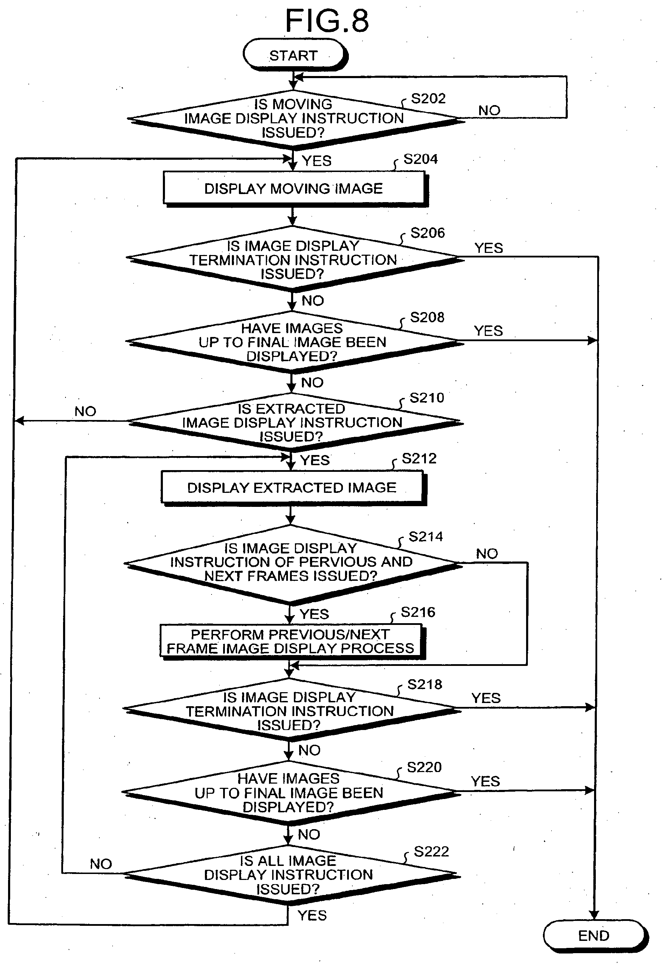

Next, an image display process procedure performed by the control unit 250 in the image display apparatus 204 will be explained with reference to Fig. 8. The control unit 250 performs a determination process to a pseudo moving image display instruction (step S202), a pseudo moving image display process (step S204), a determination process to an image display termination instruction (step S206), a determination process to a final image display (step S208), a determination process to an extraction image display instruction (step S210), and an extracted image display process (step S212), like step S102 to step S112 shown in Fig. 3.

Thereafter, the previous/next image extracting unit 253 determines whether a display instruction of an image in the previous one frame or the next one frame of the extracted image displayed in the image display area has been issued based on presence/absence of frame information inputted by the input unit 20 (step S214).

When the previous/next image extracting unit 253 determines that the display instruction of an image in the previous one frame or the next one frame of the extracted image displayed in the image display area has been issued (step S214: Yes), the display controller 52 and the previous/next image extracting unit 253 perform a previous/next frame image display process for causing the display unit 30 to display an image in the previous one frame or the one next frame of the extracted image displayed in the image display area (step S216). First, in the previous/next frame image display process, the previous/next image extracting unit 253 extracts the image in the previous one frame or the next one frame of the extracted image displayed in the image display area from the image group Pa stored in the folder F1 according to an instruction of the frame information. Thereafter, the display controller 52 causes the display unit 30 to display the image extracted by the previous/next image extracting unit 253 in the predetermined previous/next image display area.

After the previous/next frame image display process (step S216), or when the previous/next image extracting unit 253 determines that a display instruction of an image in the previous one frame or the next one frame of the extracted image displayed in the image display area has not been issued (step S214: No), the control unit 250 performs a determination process to an image display termination instruction (step S218), a determination process to a final image display (step S220), and a determination process to an all image display instruction to a pseudo moving image display (step S222), like step S114 to step S118 shown in Fig. 3.

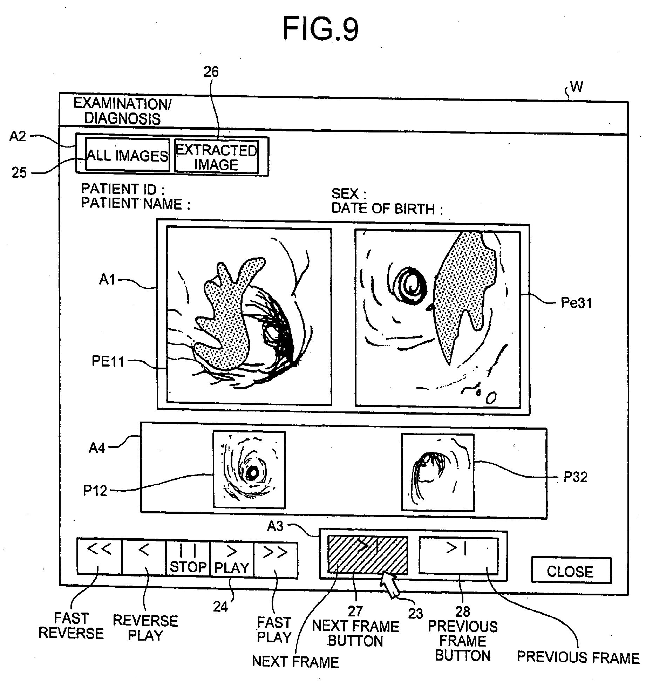

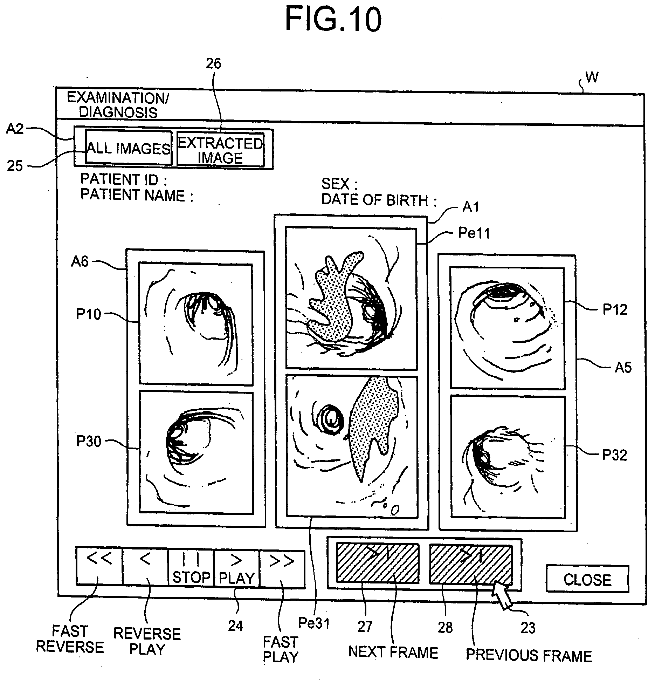

Next, a previous/next image display on a display screen in the display unit 30 will be explained with reference to Fig. 9 and Fig. 10. Fig. 9 and Fig. 10 are diagrams showing one example of a display screen in the display unit 30. A previous/next image display control area A3 and a previous/next image display area A4 in which previous and next images are displayed are further provided on the window W. The previous/next image display area A4 is displayed below the image display area A1 and the previous/next image display control area A3 is displayed below the previous/next image display area A4. A button image showing various previous/next image display control process of a next frame button 27 and a previous frame button 28 is displayed in the previous/next image display control area A3. A previous and next image corresponding to each image displayed in the image display area A1 is displayed in the previous/next image display area A4. In Fig. 9, for example, extracted images Pe11 and Pe31 are displayed in the image display area A1.

A case that images in the next one frame of the extracted images Pe11 and Pe31 are displayed as the previous and next images will be explained first with reference to Fig. 9. A user moves the cursor 23 onto the next frame button 27 and clicks the left button on the mouse. When frame information instructing display of images in the next one frame of the extracted images Pe11 and Pe31 is inputted, the previous/next image extracting unit 253 extracts an image P12 in the next one frame of the extracted image Pe11 and an image P32 in the next one frame of the extracted image Pe31 from the image group Pa stored in the folder F1 according to the instruction. The display controller 52 causes the display unit 30 to display the images P12 and P32 extracted by the previous/next image extracting unit 253 in the previous/next image display area A4 so as to correspond to display positions of the extracted images Pe11 and Pe31. Accordingly, as shown in Fig. 9, the image P12 in the next one frame of the extracted image Pe11 and the image P32 in the next one frame of the extracted image Pe31 are displayed in the previous/next image display area A4 of the window W so as to correspond to the display positions of the extracted images Pe11 and Pe31.

An image in the previous one frame of an extracted image can be displayed together with an image in the previous one frame of the extracted image. In this case, as shown in Fig. 10, a user moves the cursor 23 onto the previous frame button 28 and clicks on in a state that the next frame button 27 has been selected. When frame information instructing display of images in the previous frames of the extracted images Pe11 and Pe31 together with images in the next one frame of the extracted images Pe11 and Pe31, the previous/next image extracting unit 253 extracts an image P10 in the previous one frame of the extracted image Pe11 and an image P30 in the previous one frame of the extracted image Pe31 are extracted from the image group Pa stored in the folder F1 according to the instruction. The display controller 52 causes the display unit 30 to display the images P10 and P30 extracted by the previous/next image extracting unit 253 in a previous frame image display area A6 so as to correspond to display positions of the extracted images Pe11 and Pe31. The images P12 and P32 are displayed in a next frame image display area A5. Accordingly, as shown in Fig. 10, the images P10 and P30 in the previous one frame of the extracted images Pe11 and Pe31 are displayed in the previous frame image display area A6 provided in a left area of the image display area A1, and the images P12 and P32 in the next one frame of the extracted images Pe11 and Pe31 are displayed in the next frame image display area A5 provided in a right area of the image display area A1.

Thus, since the image display apparatus 204 according to the second embodiment displays images in the previous frame and the next frame of an extracted image or an image in either one of the frames together with the extracted image, an effect similar to that in the first embodiment can be achieved, and there can be achieved an effect that a user can rapidly confirm in what position a displayed extracted image inside a subject has been imaged.

### Third Embodiment

Next, a third embodiment will be explained. In the third embodiment, position confirmation of an extracted image inside a subject can be performed further rapidly by performing sequential display of a predetermined number of images in the previous frames or the next frames of an extracted image or displaying them in a moving image format together with the extracted image.

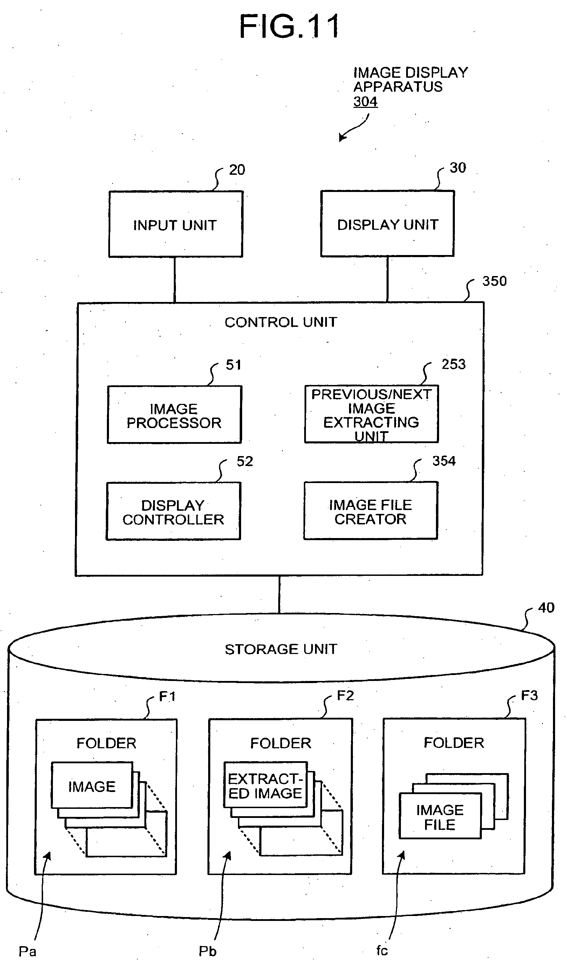

Fig. 11 is a block diagram showing a schematic configuration of an image display apparatus according to the third embodiment. As shown in Fig. 11, an image display apparatus 304 according to the third embodiment includes a control unit 350 further including an image file creator 354 in comparison with the control unit 250 according to the second embodiment. The input unit 20 inputs output instruction information instructing creation and output of a still image file or a moving image file based on an extracted image displayed in a predetermined image display area and images in a predetermined number of previous and/or next frames to a frame of the extracted image into the control unit 350. Based on the image instruction information inputted by the input unit 20, the image file creator 354 extracts images in the predetermined number of previous and/or next frames to the frame of the extracted image displayed in the predetermined image display area from the image group Pa in the folder F1 as previous and next images to create a still image file or a moving image file based on the extracted image and the extracted previous and next images. When the image file creator 354 creates the moving image file, it converts the extracted image and the previous and next images to, for example, a moving image file of an AVI format using an image processing method for moving image conversion. The image file creator 354 outputs the created image file to the storage unit 40 to store the same in a folder F3 in the storage unit 40. The display controller 52 displays the previous and next images and the extracted image in a predetermined previous/next image display area in a sequential display format or a moving image format based on the still image file or the moving image file created by the image file creator 354.

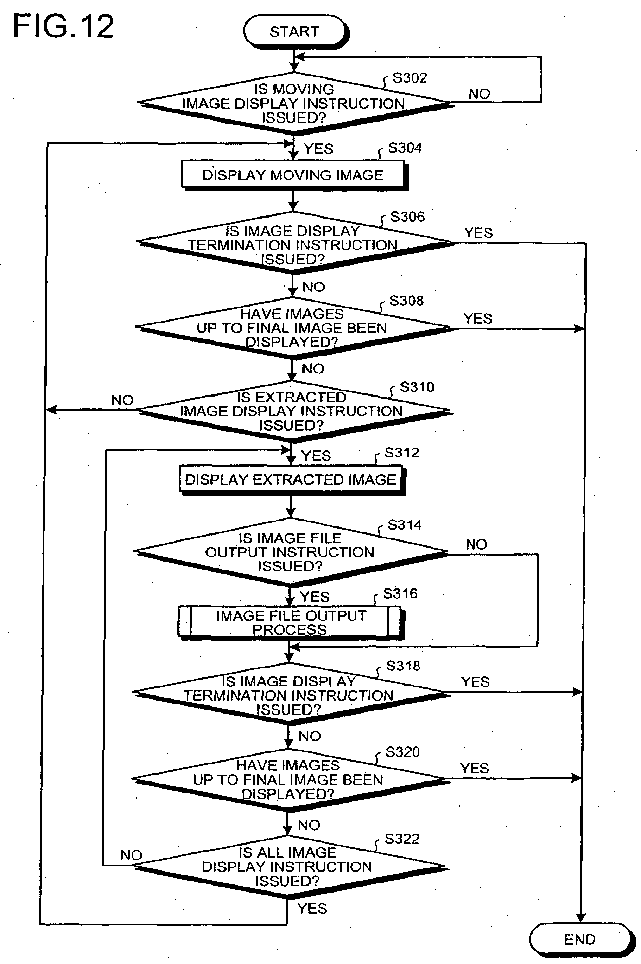

Next, an image display process procedure performed by the control unit 350 will be explained with reference to Fig. 12. In Fig. 12, the control unit 350 performs a determination process to a pseudo moving image display instruction (step S302), a pseudo moving image display process (step S304), a determination process to an image display termination instruction (step S306), a determination process to a final imaged display (step S308), a determination process to an extracted image display instruction (step S310), and an extracted image display process (step S312), like step S102 to step S112 shown in Fig. 3. Thereafter, the image file creator 354 determines whether an output instruction of an image file has been issued based on presence/absence of the output instruction information inputted by the input unit 20 (step S314).

When the image file creator 354 determines that the output instruction of the image file has been issued (step S314: Yes), the image file creator 354 and the display controller 52 create an image file including the extracted image displayed in the predetermined image display area and the images in the predetermined number of previous and/or next frames of a frame of the extracted image to perform an image file output process displaying images in the previous/next image display area based on these image files (step S316).

After the image file output process (step S316), or when the image file creator 354 determines that the output instruction of the image file has not been issued (step S314: No), the control unit 350 performs a determination process to an image display termination instruction (step S318), a determination process to a final image display (step S320), and a determination process to an all image display instruction that is the pseudo moving image display (step S322), like step S114 to step S118 shown in Fig. 3.

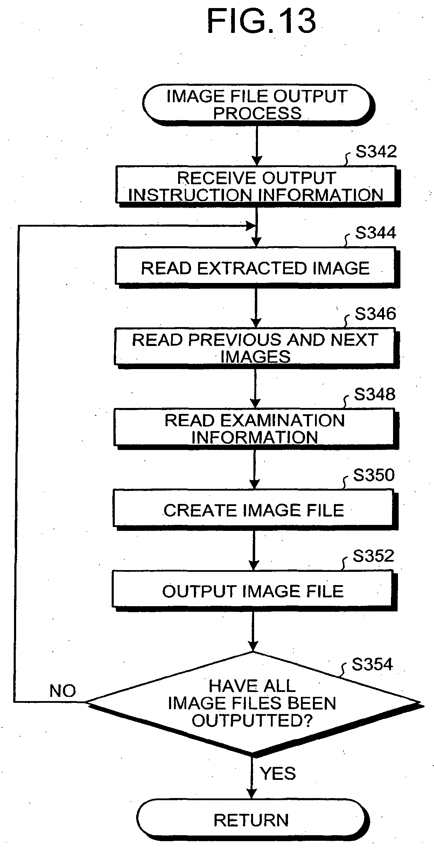

Next, a process procedure for an image file output process shown in Fig. 12 will be explained with reference to Fig. 13. In Fig. 13, first, the image file creator 354 receives the output instruction information inputted by the input unit 20 (step S342). The image file creator 354 extracts an extracted image stored in the folder F1 and reads the extracted image according to the output instruction information (step S344). In this case, the extracted image read by the image file creator 354 is an extracted image displayed in the image display area. Next, the image file creator 354 extracts images in a predetermined number of previous and/or next frames to a frame of the read extracted image from the folder F1 as the previous and next images to perform reading of the previous and next images according to the output instruction information (step S346). The image file creator 354 reads examination information that is identification information of a subject in the read extracted image and the previous and next images (step S348). For example, the examination information includes a name, an identification number, sex, and birth date of a patient who is a subject.

Thereafter, the image file creator 354 performs an image file creating process for creating a still image file or a moving image file based on the read extracted image and the previous and next images (step S350). The image file creator 354 and the display controller 52 perform an image file output process for outputting a created image file (step S352). The image file creator 354 outputs the created image file to the folder F3 in the image file output process. When the image file creator 354 creates a still image file, the display controller 52 sequentially displays the previous and next images and the extracted image in the predetermined pervious and next image display area based on the still image file. On the other hand, when the image file creator 354 creates a moving image file, the display controller 52 displays a moving image in the predetermined previous/next image display area based on the moving image file. When the image file creator 354 receives an instruction so as to output the image file and the examination information in correspondence with each other in the output instruction information, it outputs the created image file in correspondence with the read examination information to the folder F3.

Next, the control unit 350 determines whether all the image files instructed in the output instruction information has been outputted (step S354), and when it determines that all the image files have not been outputted (step S354: No), it proceeds to step S344, where the control unit 350 performs an image file creation instructed in the output instruction information, and when it is determined that all the image files have been created (step S354: Yes), the image file output process is terminated.

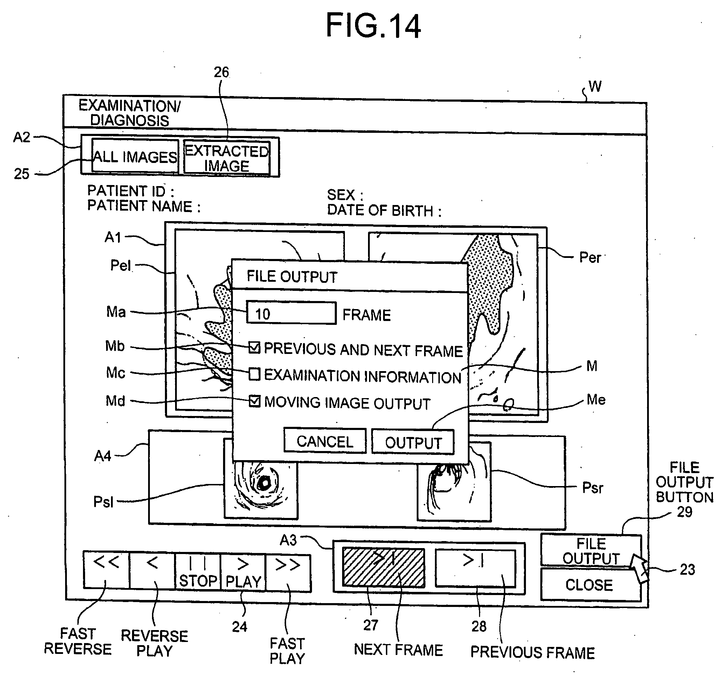

Next, a specific operation and processing will be explained with reference to Fig. 14. Fig. 14 is a diagram showing one example of a display screen of the display unit 30. As shown in Fig. 14, a file output button 29 is provided on the window W, and when the file output button 29 is selected by a left-click of the mouse, a file output window M is newly displayed and outputted. An entry item Ma of the number of frames, a selection item Mb of "previous and next frames", a selection item Mc of "examination information", and a selection item Md of "moving image output" are shown in the window M.

The number of frames entered in the entry item Ma corresponds to the number of images extracted from the folder F1 by the image file creator 354, and the selection item Mb of "previous and next frames" is an item for selecting whether the image file creator 354 extracts images in the previous and next frames of an extracted image from the folder F1. When a numeral "10" for frames is entered in the entry item Ma and the selection item Mb of "previous and next frames" is selected, the image file creator 354 extracts an extracted image displayed in the image display area, images in frames from previous one frame to previous ten frames of the extracted image, and images in frames from next one frame to next ten frames of the extracted image. When the selection item Mb of "previous and next frames" is not selected, the image file creator 354 can be set to extract either one of images in frames from previous one frame to previous ten frames of the extracted image or images in frames from next one frame to next ten frames together with the extracted image displayed in the image display area as default. The selection item Mc of "examination information" is an item for selecting whether the image file created by the image file creator 354 is output in correspondence with the examination information. The selection item Md of "moving image output" is an item for selecting whether a file format created by the image file creator 354 should be set to a moving image format. A user performs such processing as selection of each item to instruct a format of an image file to be outputted by moving the cursor 23 onto each item to input a numerical value from the keyboard and clicks of the mouse. The input unit 20 inputs output instruction information of contents instructed by a user in the control unit 350.

For example, as shown in Fig. 14, a case that, after "10 frames" is entered in the entry item Ma of the number of frames and the selection item Mb of "previous and next frames" and the selection item Md of "moving image output" are selected, the output button Me is selected will be explained. In this case, a moving image Psl corresponding to an extracted image Pe1 and a moving image Psr corresponding to an extracted image Per are respectively displayed in the previous/next image display area A4. The moving image Ps1 is displayed using a moving image file created based on twenty images corresponding to the previous ten frames and the next ten frames of an extracted image Pe1 and an image corresponding to the extracted image Pe1, while the moving image Psr is displayed using a moving image file created based on twenty images corresponding to the previous ten frames and the next ten frames of an extracted image Per and an image corresponding to the extracted image Per. When the selection item Md of "moving image output" is not selected, the twenty images corresponding to the previous ten frames and the next ten frames of the extracted image Pe1 and the extracted image Pe1 are sequentially displayed in the previous/next image display area A4 in an imaging order thereof, and the twenty images corresponding to the previous ten frames and the next ten frames of the extracted image Per and the extracted image Per are sequentially displayed thereon in an imaging order thereof.

Thus, in the third embodiment, an effect similar to those in the first and second embodiments can be achieved, and there can be achieved an effect that position confirmation of an extracted image inside a subject by a user can be made more rapidly by sequentially displaying a predetermined number of previous and/or next images to an extracted image together with the extracted image or displaying them in a moving image format.

In the first to third embodiments, the case that two images are displayed in the image display area A1 has been explained with reference to Fig. 4 to Fig. 6, Fig. 9, Fig. 10, and Fig. 14, but one image can be displayed in the image display area A1 and four images can be displayed thereon, where the number of images to be displayed is not limited, of course. The number of images to be displayed in the image display area A1 can be changed based on instruction information of the input unit 20.

In the first to third embodiments, the folder F2 storing the extracted image group Pb has been shown with reference to Fig. 2, Fig. 7, and Fig. 11.

The image display apparatuses 4, 204, and 304 explained in the first to third embodiments can be realized by performing a preliminarily prepared program in a computer system such as a personal computer or a workstation. A computer system executing an image display program having a function similar to the image display apparatuses explained in the embodiments will be explained.

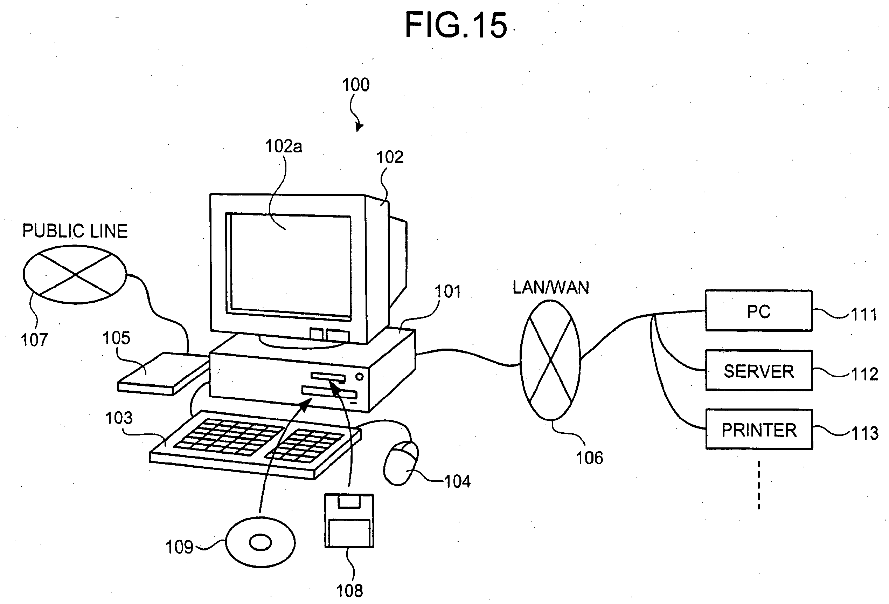

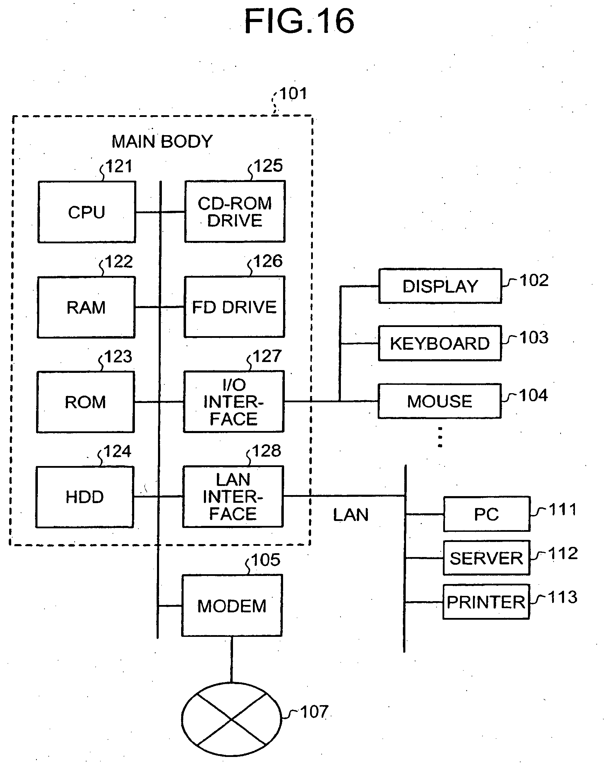

Fig. 15 is a system configuration diagram showing a configuration of a computer system using the embodiments described above. Fig. 16 is a block diagram showing a configuration of a main body in the computer system. As shown in Fig. 15, a computer system 100 according to the first to third embodiments includes a main body 101, a display 102 for displaying information such as images on a display screen 102a according to an instruction from the main body 101, a keyboard 103 for inputting various information into the computer system 100, and a mouse 104 for indicating arbitrary position on the display screen 102a of the display 102.

As shown in Fig. 16, the main body 101 in the computer system 100 includes a CPU 121, a RAM 122, a ROM 123, a hard disk drive (HDD) 124, a CD-ROM drive 125 that accommodates a CD-ROM 109, an FD drive 126 that accommodates a flexible disk (FD) 108, an I/O interface 127 connecting the display 102, the keyboard 103, and the mouse 104, and a LAN interface 128 connected to a local area network or a wide area network (LAN/WAN) 106.

The computer system 100 is connected with a modem 105 connected to a public line 107 such as the Internet and is connected with another computer system (PC) 111, a server 112, a printer 113, and the like via the LAN interface 128 and the LAN/WAN 106.

The computer system 100 realizes an image display apparatus by reading and executing an image display program recorded in a predetermined recording medium. The predetermined recording medium includes any recording medium recording an image display program that can be read by the computer system 100, including "portable physical medium" such as a flexible disk (FD) 108, the CD-ROM 109, an MO disc, a DVD disc, a magnetic optical disc, or an IC card, and also "fixed physical medium" such as the hard disk drive (HDD) 124, the RAM 122, or the ROM 123 provided outside or inside the computer system 100, and "communication medium" saving a program for a short period of time during transmission of a program, such as the public line 107 connected via the modem 105 or the LAN/WAN 106 connected with another computer system 111 and another server 112.

That is, the image display program is recorded on a recording medium such as the "potable physical medium", "fixed physical medium", and "communication medium" in a computer-readable manner, and the computer system 100 can realize an image display apparatus and an image display method by reading the image display program from such a recording medium and executing the same. The image display program is not limited to the one executed by the computer system 100, but similarly applied to a case that another computer system 111 or the server 112 executes the image display program, or to a case that these computers execute, the image display program in cooperation with each other.

### INDUSTRIAL APPLICABILITY

As described above, the image display apparatus, the image display method, and the image display program according to the present invention are useful for a medical observing apparatus that is inserted into the inside of a human body to observe an examined site, and they are particularly suitable for enabling rapid image confirmation by a user.

## Claims

1. An image display apparatus (4; 204; 304) including a display unit (30) configured to sequentially display a plurality of stored images (Pa) in an imaging order thereof, comprising
a control unit (50; 250; 350) configured to cause the display unit (30) to display an extracted image (Pb; Pe11; Pe31) preliminarily extracted from the plurality of stored images (Pa) under a predetermined extraction condition;
further comprising an input unit (20) configured to input image instruction information for designating either one of the plurality of stored images (Pa) or the extracted image (Pb; Pe11; Pe31) as an image to be displayed on the display unit (30), wherein
the control unit (50; 250; 350) is configured to switch an image displayed in a predetermined display area (A1) by the display unit (30) to an image designated according to the image instruction information, and to cause the display unit (30) to display the switched image in the predetermined display area (A1);
wherein the plurality of stored images (Pa) are images taken by imaging inside of a subject (1);
wherein the predetermined extraction condition represents that an abnormal part in the subject (1) has been imaged;
**characterised in that** the image display apparatus further comprises a storage unit 40, in which the plurality of stored images (Pa) and the extracted image (Pb; Pe11; Pe31) are stored in folders F1 and F2, respectively;
wherein the control unit (50; 250; 350) is configured to extract images in a predetermined number of previous and/or next frames to the extracted image (Pb; Pe11, Pe31) displayed in the predetermined display area (A1) from the plurality of stored images (Pa) as previous and next images (P10, P12, P30, P32), and to cause the display unit (30) to display the previous and next images (P10, P12, P30, P32) together with the extracted image (Pb; Pe11, Pe31).

2. The image display apparatus according to claim 1, wherein the control unit creates a still image file based on the previous and next images and the extracted image that corresponds to the previous and next images, and outputs the still image file.

3. The image display apparatus according to claim 1, wherein the control unit creates a moving image file based on the previous and next images and the extracted image that corresponds to the previous and next images, and outputs the moving image file.

4. The image display apparatus according to claim 2 or 3, wherein the control unit outputs identification information of the subject in the extracted image and the previous and next images so as to correspond to the still image file or the moving image file.

5. The image display apparatus according to claim 1, wherein the abnormal part is a bleeding part.

6. An image display method for sequentially displaying a plurality of stored images in an imaging order thereof, using an apparatus according to any one of claims 1-5, the method comprising
an input step of inputting image instruction information designating either one of the plurality of stored images (Pa) or an extracted image (Pb; Pe11, Pe31) preliminarily extracted from the plurality of stored images (Pa) under a predetermined extraction condition (S110; S210; S310);
a switching step of switching an image displayed in a predetermined display area (A1) of a display unit (30) to an image designated by the image instruction information (S112; S212; S312); and
a display step of displaying the switched image in the predetermined display area (A1); further comprising
an extracting step of extracting images in a predetermined number of previous and/or next frames to a frame of the extracted image (Pb; Pe11, Pe31) displayed in the predetermined display area (A1) from the plurality of stored images (Pa) as previous and next images (P10, P12, P30, P32), wherein
the display step displays the previous and next images (P10, P12, P30, P32) together with the extracted image (Pb: Pe11, Pe31).

7. The image display method according to claim 6, comprising an image file output step of creating a still image file or a moving image file based on the previous and next images extracted at the extracting step and the extracted image that corresponds to the previous and next images, and of outputting the still image file or the moving image file.

8. A computer readable recording medium **characterized by** having a program for the computer to execute the method as described in any one of claims 6 to 7 stored thereon, when run on an apparatus according to any one of claims 1-5.

## Patentansprüche

1. Bildanzeigegerät (4; 204; 304), mit einer Anzeigeeinheit (30), die dazu eingerichtet ist, nacheinander eine Mehrzahl von gespeicherten Bildern (Pa) in ihrer Bildaufnahmereihenfolge anzuzeigen, das
eine Steuereinheit (50; 250; 350) umfasst, die dazu eingerichtet ist, die Anzeigeeinheit (30) zu veranlassen, ein extrahiertes Bild (Pb; Pe11; Pe31) anzuzeigen, das vorab unter einer vorbestimmten Extraktionsbedingung aus der Mehrzahl von gespeicherten Bildern (Pa) extrahiert worden ist;
ferner eine Eingabeeinheit (20) umfasst, die dazu eingerichtet ist, eine Bildbefehlsinformation einzugeben, um entweder eines der Mehrzahl von gespeicherten Bildern (Pa) oder das extrahierte Bild (Pb; Pe11; Pe31) als ein auf der Anzeigeeinheit (30) anzuzeigendes Bild zu bestimmen, wobei
die Steuereinheit (50; 250; 350) dazu eingerichtet ist, ein durch die Anzeigeeinheit (30) in einem vorbestimmten Anzeigebereich (A1) angezeigtes Bild in ein Bild zu wechseln, das gemäß der Bildbefehlsinformation bestimmt worden ist, und die Anzeigeeinheit (30) zu veranlassen, das gewechselte Bild in dem vorbestimmten Anzeigebereich (A1) anzuzeigen;
wobei die Mehrzahl von gespeicherten Bildern (Pa) Bilder sind, die durch Bildaufnahme im Inneren eines Subjekts (1) aufgenommen worden sind;
wobei die vorbestimmte Extraktionsbedingung repräsentiert, dass ein abnormales Teil in dem Subjekt (1) abgebildet worden ist;
**dadurch gekennzeichnet, dass** das Bildanzeigegerät ferner eine Speichereinheit (40) umfasst, in der die Mehrzahl von gespeicherten Bildern (Pa) und das extrahierte Bild (Pb; Pe11; Pe31) in Foldern F1 bzw. F2 gespeichert sind;
wobei die Steuereinheit (50; 250; 350) dazu eingerichtet ist, aus der Mehrzahl von gespeicherten Bildern (Pa) Bilder in einer vorbestimmten Anzahl von Frames, die dem in dem vorbestimmten Anzeigebereich (A1) angezeigten extrahierten Bild (Pb; Pe11; Pe31) vorangehen und/oder nachfolgen, als vorangehende und nachfolgende Bilder (P10, P12, P30, P32) zu extrahieren und die Anzeigeeinheit (30) zu veranlassen, die vorangehenden und nachfolgenden Bilder (P10, P12, P30, P32) gemeinsam mit dem extrahierten Bild (Pb; Pe11; Pe31) anzuzeigen.

2. Bildanzeigegerät gemäß Anspruch 1,
bei dem die Steuereinheit auf der Basis der vorangehenden und nachfolgenden Bilder und des extrahierten Bilds, das den vorangehenden und nachfolgenden Bildern entspricht, eine Standbilddatei erzeugt und die Standbilddatei ausgibt.

3. Bildanzeigegerät gemäß Anspruch 1,
bei dem die Steuereinheit auf der Basis der vorangehenden und nachfolgenden Bilder und des extrahierten Bilds, das den vorangehenden und nachfolgenden Bildern entspricht, eine Bewegtbilddatei erzeugt und die Bewegtbilddatei ausgibt.

4. Bildanzeigegerät gemäß Anspruch 2 oder 3,
bei dem die Steuereinheit eine Identifikationsinformation des Subjekts in dem extrahierten Bild und den vorangehenden und nachfolgenden Bildern ausgibt, um der Standbilddatei oder der Bewegtbilddatei zu entsprechen.

5. Bildanzeigegerät gemäß Anspruch 1,
bei dem das abnormale Teil ein blutendes Teil ist.

6. Bildanzeigeverfahren zum aufeinanderfolgenden Anzeigen einer Mehrzahl von gespeicherten Bildern in ihrer Bildaufnahmereihenfolge unter Verwendung eines Geräts gemäß einem der Ansprüche 1 bis 5, wobei das Verfahren umfasst einen Eingabeschritt zum Eingeben einer Bildbefehlsinformation, die entweder eines der Mehrzahl von gespeicherten Bildern (Pa) oder ein extrahiertes Bild (Pb; Pe11; Pe31), das vorab unter einer vorbestimmten Extraktionsbedingung (S110; S210; S310) aus der Mehrzahl von gespeicherten Bildern (Pa) extrahiert worden ist, bestimmt,
einen Wechselschritt zum Wechseln eines in einem vorbestimmten Anzeigebereich (A1) einer Anzeigeeinheit (30) angezeigten Bilds in ein Bild, das durch die Bildbefehlsinformation (S112; S212; S312) bestimmt worden ist; und
einen Anzeigeschritt zum Anzeigen des gewechselten Bilds in dem vorbestimmten Anzeigebereich (A1);
und das ferner einen Extraktionsschritt zum Extrahieren von Bildern in einer vorbestimmten Anzahl von Frames, die einem Frame des in dem vorbestimmten Anzeigebereich (A1) angezeigten extrahierten Bild (Pb; Pe11; Pe31) vorangehen und/oder nachfolgen, als vorangehende und nachfolgende Bilder (P10, P12, P30, P32) aus der Mehrzahl von gespeicherten Bildern (Pa), wobei
der Anzeigeschritt die vorangehenden und nachfolgenden Bilder (P10, P12, P30, P32) gemeinsam mit dem extrahierten Bild (Pb; Pe11; Pe31) anzeigt.

7. Bildanzeigeverfahren gemäß Anspruch 6,
das einen Bilddateiausgabeschritt zum Erzeugen einer Standbilddatei oder einer Bewegtbilddatei auf der Basis der in dem Extraktionsschritt extrahierten vorangehenden und nachfolgenden Bilder und des extrahierten Bilds, das den vorangehenden nachfolgenden Bildern entspricht, und zum Ausgeben der Standbilddatei oder der Bewegtbilddatei umfasst.

8. Computerlesbares Speichermedium, **dadurch gekennzeichnet, dass** es ein darauf gespeichertes Programm für den Computer zur Ausführung des in einem der Ansprüche 6 bis 7 beschriebenen Verfahrens umfasst, wenn es auf einem Gerät gemäß einem der Ansprüche 1 bis 5 ausgeführt wird.

## Revendications

1. Appareil d'affichage d'images (4 ; 204 ; 304) incluant une unité d'affichage (30) configurée pour afficher séquentiellement une pluralité d'images stockées (Pa) dans un ordre d'imagerie de celles-ci, comprenant
une unité de commande (50 ; 250 ; 350) configurée pour faire en sorte que l'unité d'affichage (30) affiche une image extraite (Pb ; Pe11; Pe31) préliminairement extraite de la pluralité d'images stockées (Pa) sous une condition d'extraction prédéterminée ;
comprenant en outre une unité d'entrée (20) configurée pour entrer des informations d'instruction d'images pour désigner l'une ou l'autre de la pluralité d'images stockées (Pa) ou de l'image extraite (Pb ; Pe11; Pe31) comme une image devant être affichée sur l'unité d'affichage (30), dans lequel
l'unité de commande (50 ; 250 ; 350) est configurée pour commuter une image affichée dans une zone d'affichage (A1) prédéterminée par l'unité d'affichage (30) sur une image désignée conformément aux informations d'instruction d'images, et pour faire en sorte que l'unité d'affichage (30) affiche l'image commutée dans la zone d'affichage (A1) prédéterminée ;
dans lequel la pluralité d'images stockées (Pa) sont des images prises par imagerie à l'intérieur d'un sujet (1) ;
dans lequel la condition d'extraction prédéterminée représente qu'une partie anormale dans le sujet (1) a été imagée ;
**caractérisé en ce que** l'appareil d'affichage d'images comprend en outre
une unité de stockage (40), dans laquelle la pluralité d'images stockées (Pa) et l'image extraite (Pb ; Pe11; Pe31) sont stockées dans des dossiers F1 et F2, respectivement ;
dans lequel l'unité de commande (50 ; 250 ; 350) est configurée pour extraire des images dans un nombre prédéterminé de trames précédentes et/ou suivantes par rapport à l'image extraite (Pb ; Pe11; Pe31) affichée dans la zone d'affichage (A1) prédéterminée parmi la pluralité d'images stockées (Pa) comme des images précédentes et suivantes (P10, P12, P30, P32), et pour faire en sorte que l'unité d'affichage (30) affiche les images précédentes et suivantes (P10, P12, P30, P32) en même temps que l'image extraite (Pb ; Pe11; Pe31).

2. Appareil d'affichage d'images selon la revendication 1, dans lequel l'unité de commande crée un fichier d'images fixes sur la base des images précédentes et suivantes et de l'image extraite qui correspond aux images précédentes et suivantes, et délivre en sortie le fichier d'images fixes.

3. Appareil d'affichage d'images selon la revendication 1, dans lequel l'unité de commande crée un fichier d'images animées sur la base des images précédentes et suivantes et de l'image extraite qui correspond aux images précédentes et suivantes, et délivre en sortie le fichier d'images animées.

4. Appareil d'affichage d'images selon la revendication 2 ou 3, dans lequel l'unité de commande délivre en sortie des informations d'identification du sujet dans l'image extraite et les images précédentes et suivantes de façon à ce qu'elles correspondent au fichier d'images fixes ou au fichier d'images animées.

5. Appareil d'affichage d'images selon la revendication 1, dans lequel la partie anomale est une partie saignante.

6. Procédé d'affichage d'images pour afficher séquentiellement une pluralité d'images stockées dans un ordre d'imagerie de celles-ci, en utilisant un appareil selon l'une quelconque des revendications 1 à 5, le procédé comprenant
une étape d'entrée pour entrer des informations d'instruction d'images désignant l'une ou l'autre de la pluralité d'images stockées (Pa) ou d'une image extraite (Pb ; Pe11; Pe31) préliminairement extraite de la pluralité d'images stockées (Pa) sous une condition d'extraction prédéterminée (S110 ; S210 ; S310) ;
une étape de commutation pour commuter une image affichée dans une zone d'affichage (A1) prédéterminée d'une unité d'affichage (30) sur une image désignée par les informations d'instruction d'images (S112 ; S212 ; S312) ; et
une étape d'affichage pour afficher l'image commutée dans la zone d'affichage (A1) prédéterminée ;
comprenant en outre
une étape d'extraction pour extraire des images dans un nombre prédéterminé de trames précédentes et/ou suivantes par rapport à une trame de l'image extraite (Pb ; Pe11; Pe31) affichée dans la zone d'affichage (A1) prédéterminée parmi la pluralité d'images stockées (Pa) comme des images précédentes et suivantes (P10, P12, P30, P32), dans lequel
l'étape d'affichage affiche les images précédentes et suivantes (P10, P12, P30, P32) en même temps que l'image extraite (Pb ; Pe11; Pe31).

7. Procédé d'affichage d'images selon la revendication 6, comprenant une étape de sortie de fichier d'images pour créer un fichier d'images fixes ou un fichier d'images animées sur la base des images précédentes et suivantes extraites à l'étape d'extraction et de l'image extraite qui correspond aux images précédentes et suivantes, et pour délivrer en sortie le fichier d'images fixes ou le fichier d'images animées.

8. Support d'enregistrement lisible par un ordinateur **caractérisé en ce qu'**ayant un programme pour que l'ordinateur exécute le procédé selon l'une quelconque des revendications 6 à 7 stocké sur celui-ci, lorsqu'il tourne sur un appareil selon l'une quelconque des revendications 1 à 5.
